# EUROPEAN PATENT APPLICATION

(11) **EP 4 006 456 A1**
(43) Date of publication of application: **01.06.2022**
(21) Application number: 20210703.3
(22) Date of filing: 30.11.2020
(51) Int. Cl.: F25C 1/243, A61J 9/00, A61M 1/06, B65D 85/80

(54) **MILK STORAGE CONTAINER**

(71) Applicant: Koninklijke Philips N.V., 5656 AG Eindhoven (NL)
(72) Inventor: CERULLO, Alfredo, 5656 AE Eindhoven (NL); VAN ROOSMALEN, Elisabeth Johanna, 5656 AE Eindhoven (NL); BROCKHUIS, Lili-Marjan, 5656 AE Eindhoven (NL); LIPSCH, Job, 5656 AE Eindhoven (NL); BARENDREGT, Nicole, 5656 AE Eindhoven (NL); DE VREEDE, Jasper, 5656 AE Eindhoven (NL); PAZOOKI, Amir Masoud Akbari, 5656 AE Eindhoven (NL)
(74) Representative: Philips Intellectual Property & Standards

(57) **Abstract**

A milk storage container (20) for use with a breast pump, comprising: a head (26) for attachment to a breast pump (10), the head having an opening (50) for receiving milk from the breast pump; and a base part (21) which defines a plurality of individual compartments (22) and a channel arrangement (24) leading from the opening (50) to the compartments (22), wherein the compartments (22) are formed of a rigid or semi-rigid material and wherein frozen milk is removable individually from the compartments.

## Description

### FIELD OF THE INVENTION

This invention relates to a milk storage container for freezing milk.

### BACKGROUND OF THE INVENTION

Breast milk may be stored in a refrigerator for a limited period of time, such as for four days. Breast milk may also be frozen and stored in a freezer, such as for up to six months. This invention relates in particular to the storage of expressed breast milk in a freezer.

Most mothers who express do so to make sure there is always enough milk for their child, and for this reason mothers often build up stock for future use. Freezing breast milk is one of the best ways to preserve milk for a longer period of time, but after thawing the milk needs to be consumed within 24 hours.

Typically, breast milk is frozen in bottles or in breast milk bags. The downside of regular bottles and breast milk bags is the fact that a large amount of milk is frozen, which needs to be thawed all at once and consumed within 24 hours. Not managing this correctly results in waste due to spoilage.

For this reason, some mothers use ice cube trays. The mother for example pours their freshly expressed milk from a bottle or storage vessel which they used to express the milk into the tray, and then stores the frozen tray in a zip lock bag. When transferring milk to the tray, some milk will always be wasted due to the fatty and sticky nature of breast milk. The process of pouring the milk into the ice cube tray (or freezer bags) also frequently results in more spoilage.

US 2009/088684 discloses a breast milk freezer bag having multiple receiving units which, once frozen, can be individually broken off and thawed. The freezer bag is not reusable because it is broken up in use. The bag needs to be fitted in a receiving container to enable it to be connected to a breast pump, giving rise to a number of components, which is inconvenient for the user.

Furthermore, once one of the portions of frozen milk units is removed from the breast milk freezer bag, the container is broken, and the remaining frozen milk will be in contact with air in the freezer. This poses a risk to the quality and safety of the breast milk stored.

There is therefore a need for an improved solution to the storage of breast milk to allow only a desired quantity to be thawed at a time.

### SUMMARY OF THE INVENTION

The invention is defined by the claims.

According to examples in accordance with an aspect of the invention, there is provided a milk storage container for use with a breast pump, comprising:
a head for attachment to a breast pump having an opening for receiving milk from the breast pump; and
a base part which defines a plurality of individual compartments and a channel arrangement leading from the opening to the compartments,
wherein the compartments are formed of a rigid or semi-rigid material and wherein frozen milk is removable individually from the compartments.

This container enables individual compartments to be filled during use of a breast pump, so that the milk can be frozen in individualized compartments. The base part is rigid or semi-rigid, by which is meant the base part is sufficiently rigid to hold its shape, and hence have a same shape before and after filling. This means the base part can easily be cleaned and sterilized for re-use. It means there are few parts, and no waste. The removal of the frozen milk portions is without damage to the container so that it can be reused.

The compartments are sufficiently rigid that they retain their shape when empty, and thus may be washed easily and re-used. The base part does not collapse when empty.

The container is connectable directly to the breast pump, without use of a bottle or other container. The container can preferably be opened and re-sealed after removal of one or multiple portions of frozen breast milk.

The head may be formed of the same material as the base part (and indeed it may be integrated with it), or if it is a separate part it may be more rigid than the base, for example rigid plastic. The head is for example a threaded head, but other types of connection may be used.

The compartments are for example sufficiently individually deformable to assist removal of frozen milk from individual compartments. By deforming a compartment, for example by deforming a base beneath the frozen milk in the compartment, an individual milk portion can be removed from the base.

The container may further comprise a removable cover for covering the base part. The base part may define the compartments and the cover then closes the tops of the compartments. This defines a tray type design, with a tray of compartments and a lid.

The base part may comprise an array of cuboid shaped individual compartments arranged in a plane, wherein the plane is parallel to a direction of attachment of the head to the breast pump, which attachment direction is for example a rotation axis of a threaded head. This for example defines an upright planar array of compartments, with the head at the top.

The removable cover and the base part may each comprise compartment portions and channel portions which together define the channel arrangement and the plurality of individual compartments.

When the cover is removed, the frozen milk portions will stand proud from the base portion so they can be picked out individually. The base portion and the cover may in this case be rigid, or the base portion may still be deformable to assist removal of the milk portions.

The head and the base part may comprise a unitary piece. This reduces the number of components. The unitary piece may be silicone.

The head may be detachable from the base part. This allows the head to be formed of a rigid material for example most suitable for forming a connector such as a threaded connector, and the base part to be formed of a material most suitable for the compartments. The base part may then be silicone, and the head may be a rigid plastic.

Instead of the tray type design defined above, the base part may comprise a cup, and the container further comprises a divider for dividing the cup into the individual compartments.

This design is more similar to a standard bottle, but with the interior divided into compartments. The divider is for example silicone.

The compartments may comprise segments of a cylinder. The cup is thus a cylindrical shape and the divider forms segments. Each segment is sized to fit through the opening of a compatible feeding bottle.

The head may form a removable cover to the cup. The head thus functions both to close the top of the cup (but still with an opening for transferring milk to the compartments) and to form the thread for connection to the breast pump. The container may further comprise a cap for closing the opening.

In this cup type design, the base part may be vertically stackable over an identical base part, such that a bottom of one base part functions as a closure lid for a base part beneath. A single cover and cap may be provided over the top base part of the stack.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a container attached to a breast pump;
Figure 2 shows the design of Figure 1 in side view;
Figure 3 shows a variation to the design of Figure 2;
Figure 4 shows second example of container;
Figure 5 shows a third example of container;
Figure 6 shows an exploded view of the design of Figure 5.
Figure 7 shows one cup base and the threaded part forming a lid for the design of Figure 5;
Figure 8 shows a stacked arrangement of three base parts; and
Figure 9 shows how one frozen milk portion fits through the top opening of a standard milk feeding bottle.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a milk storage container for use with a breast pump which has a head for attachment to a breast pump and a base part which defines an array of individual compartments. The compartments are rigid or semi-rigid so that they can easily be cleaned and sterilized and frozen milk is removable individually from the compartments.

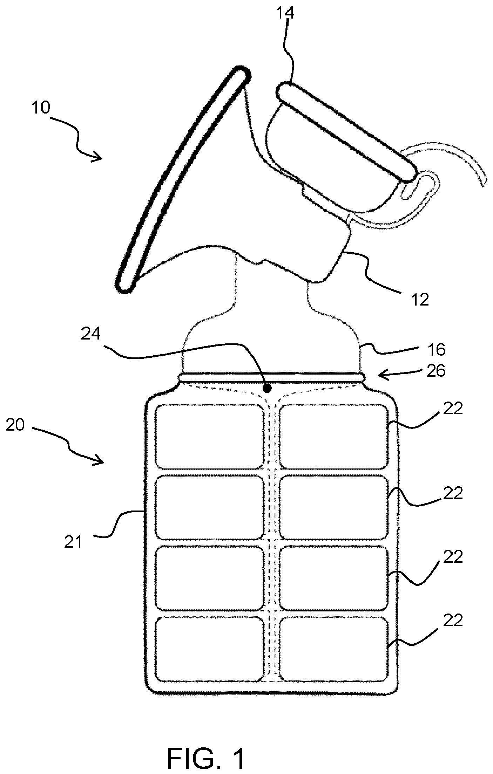

Figure 1 shows a first example of a container 20 attached to a breast pump 10. The breast pump is entirely conventional and comprises an expression kit 12, a vacuum pump 14 and a connection interface 16 for connecting the breast pump to a collection vessel. The vacuum pump may be electric as shown or it may be manual. The pump may also be at the location of the expression kit, or it may be more remote with a tube coupling between them. Conventionally, the collection vessel is typically a feeding bottle.

This invention relates to a different container design to function as the collection vessel, one example of which is shown in Figure 1.

The collection vessel 20 functions as milk storage container for placing in the freezer directly after being detached from the breast pump.

The container 20 comprises a base part 21 and a head 26 for attachment to the breast pump 10. In this example, the head is a threaded head having an opening for receiving milk from the breast pump. However, other connections between the container and the breast pump may be used.

The base part 21 defines an array of individual compartments 22 and a channel arrangement 24 leading from the head 26, in particular from the opening of the head, to the compartments 22.

The compartments in this example are cuboid shaped and they are arranged in a plane. The plane is parallel to a direction of attachment of the head to the breast pump, for example parallel to a rotation axis of the head 26 when it is a threaded head. This for example defines an upright planar array of compartments, with the head at the top.

The compartments are formed of a rigid or semi-rigid material. They may be formed in a rigid material such as plastic or a semi-rigid material such as silicone. In either case, frozen milk is removable individually from the compartments 22 without damaging the base part 21. This may be achieved by deformation of the base part 21 for example by pushing up from beneath the compartment if it is silicone, or by bending or twisting the overall structure if it is a more rigid plastic, in the same way that ice cubes are released from an ice cube tray.

The individual compartments 22 are filled during use of the breast pump, so that the milk is frozen in individualized compartments simply by transferring the container to the freezer. The base part can easily be cleaned and sterilized for re-use once empty, so that there are few parts, and no waste.

The container 20 is connectable directly to the breast pump, without use of a bottle or other container.

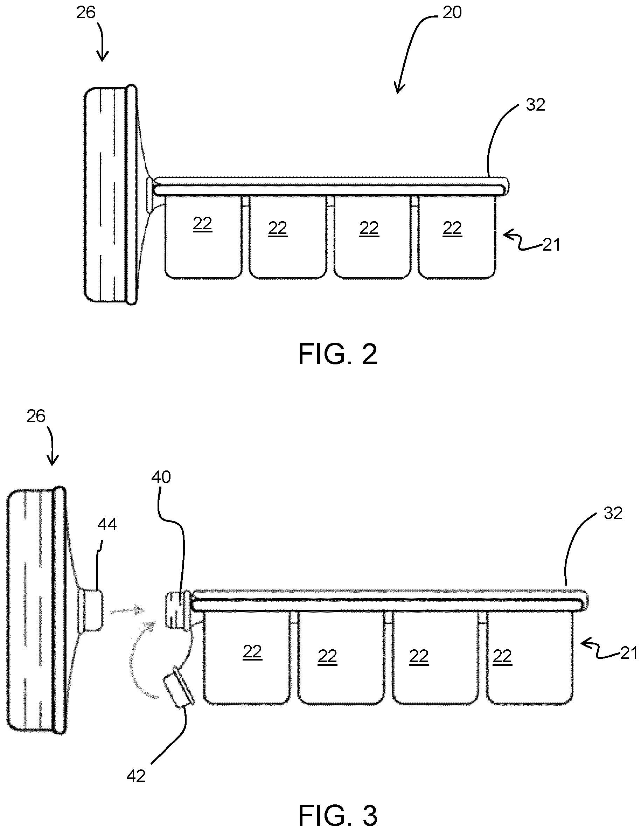

Figure 2 shows the design of Figure 1 in side view. In this design, the head 26 is formed of the same material as the base part 21 and they are integrated as a single molded part. The single part may be silicone or plastic.

Figure 2 also shows a cover 32 which is applied over the base part 21 to close the compartments 22. The cover is removed to provide access to the compartments, and individual frozen milk portions can then be removed. The base part 21 defines the compartments and the cover 32 closes the tops of the compartments. This defines a tray type design, with a tray of compartments and a lid.

Figure 3 shows a variation to the design of Figure 2 in which the head 26 is removable from the base part 21 so that the volume of the container that is placed into the freezer is reduced. The head may be made of the same material as the base part or it may be different, for example a more rigid plastic suitable for forming the coupling to the breast pump. The base part 21 has a first fitting 40 and the head 26 has a second fitting 44. These may form a screw coupling or a push fit coupling. A cap 42 enables the opening through the first fitting to be closed.

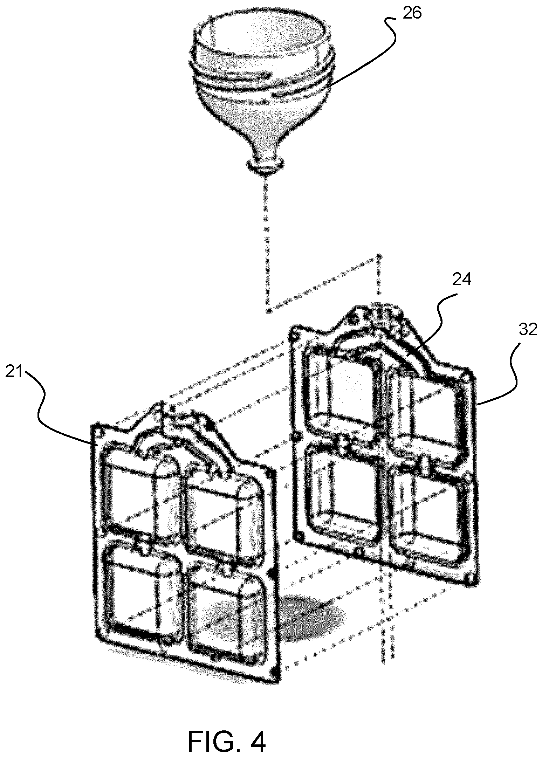

Figure 4 shows another example in which the base part 21 and the removable cover 32 each comprise compartment portions and channel portions which together define the channel arrangement 24 and the array of individual compartments. When the cover 32 is removed, the frozen milk portions will stand proud from the base portion so they can be picked out individually.

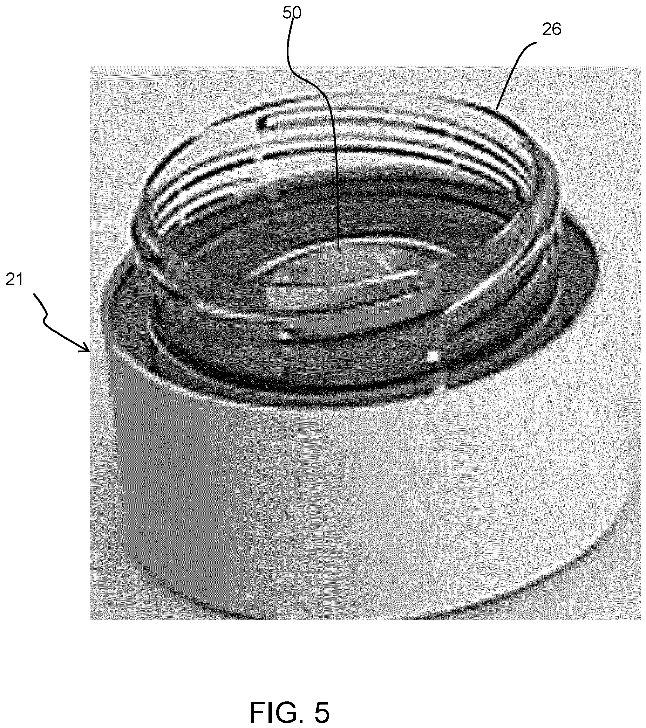

Instead of the tray type design described above, the base part 21 may comprise a cup (or bottle) type container as shown in Figure 5. The head 26 then also forms a lid to the cup. There is an opening 50 of the head communicating with the channel arrangement.

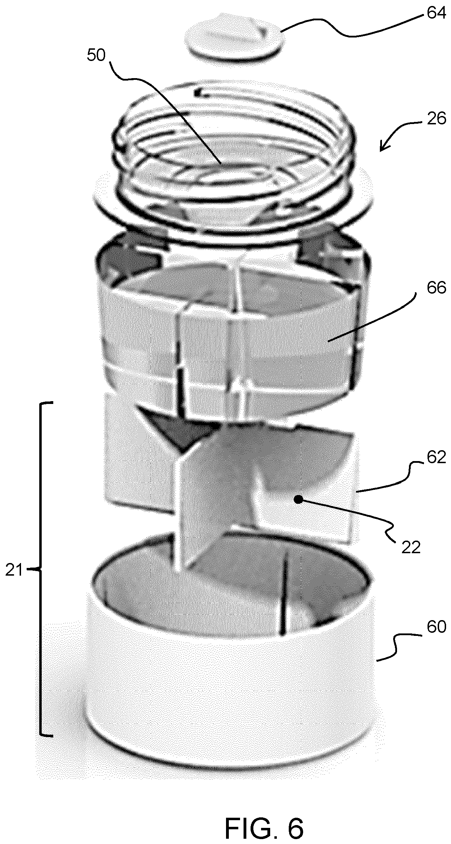

Figure 6 shows an exploded view of the design of Figure 5.

The base part 21 comprises the cup base 60 and a divider 62 for dividing the cup into the individual compartments 22. This design is more similar to a standard bottle, but with the interior divided into compartments. The cup base 60 and the divider 62 are for example silicone. The head 26 is for example a more rigid plastic.

The divider comprises a set of vertical and radially extending leaves so that the compartments may comprise segments of a cylinder. The cup is thus a cylindrical shape and the divider forms the segments. The resulting frozen milk portions 66 comprise segments, and each one is sized to fit through the opening of a compatible feeding bottle.

The head 26 forms a removable cover to the cup base 60. The head thus functions both to close the top of the cup base 60 and to form the thread for connection to the breast pump. The container further comprises a cap 64 for closing the opening 50. The cap is for example a silicone plug.

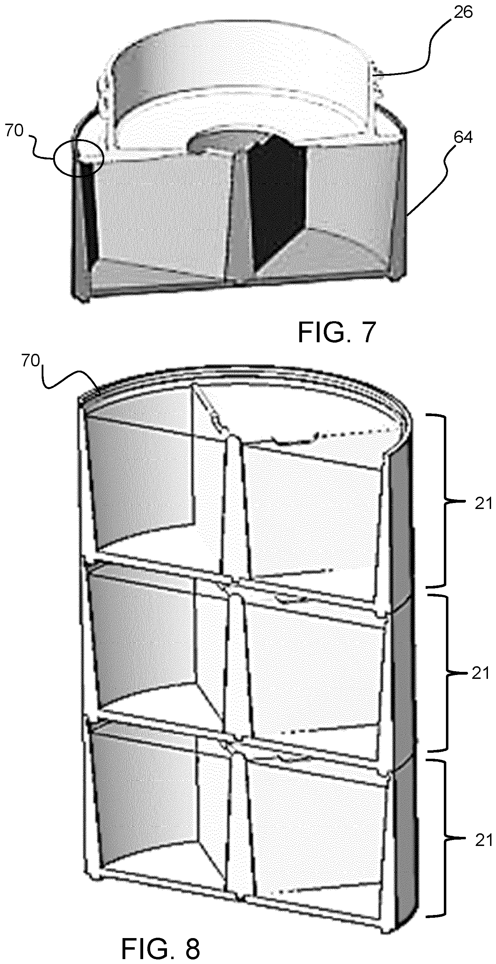

Figure 7 shows one cup base 64 and a threaded head part 26 forming a lid.

The threaded head part for example screws into the cup base 64 by a pair of engaging threads 70. However, the threaded head part may instead be a push fit into or over the cup base.

In this cup type design, the base part may be vertically stackable over an identical base part, such that a bottom of one base part functions as a closure lid for a base part beneath.

Figure 8 shows a stacked arrangement of three base parts 21. A single cover and cap may be provided over the top base part of the stack. The bottom of each base part in this example has a threaded ring so that one base part may be screwed onto the top of the base part beneath, in the stack. The bases may instead stack simply by a push fit coupling between them.

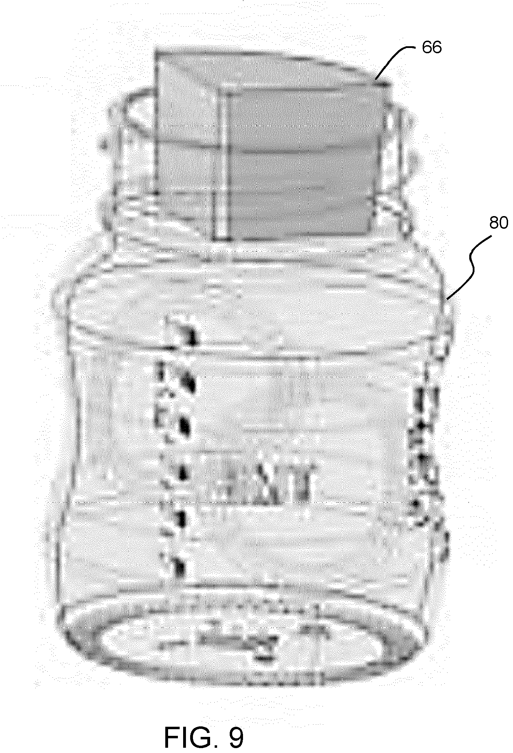

Figure 9 shows how one frozen milk portion 66 fits through the top opening of a standard milk feeding bottle 80.

The invention thus provides a portioning solution similar to ice cube freezer bags or trays, but which can be directly attached to the expression kit of a breast pump. While expressing, the milk directly flows into the container and the milk is spread across the multiple compartments, portioning the milk in smaller sizes. The container can then be placed into the freezer and each individual portion can be removed while frozen, and thawed individually.

The channel arrangement may fill the compartments one at a time (e.g. the lowest first) or in parallel. If filled one by one, some compartments can be full to capacity even when the overall container is not full. Once filled, all compartments typically hold the same volume such as 15ml, but there could equally be compartments of different volumes. The channel arrangement may for example be asymmetrical so that a single compartment is filled before the liquid spills over to the next. This leads to more compartments with exactly the determined volume.

The examples above include compartments with a cuboid shape as well as compartments with a pie-shaped wedge shape. However, other compartment shapes are possible such as cylindrical or spherical.

An example with stackable full milk compartments is shown above. Multiple base parts may also be stackable when empty in a more compact way, with the compartments of one base part fitting inside the compartments of another. For example the compartments may be tapered to all them to stack when empty, to reduce storage space. The lids may then stack separately.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A milk storage container (20) for use with a breast pump, comprising:
a head (26) for attachment to a breast pump (10), the head having an opening (50) for receiving milk from the breast pump; and
a base part (21) which defines a plurality of individual compartments (22) and a channel arrangement (24) leading from the opening (50) to the compartments (22),
wherein the compartments (22) are formed of a rigid or semi-rigid material and wherein frozen milk is removable individually from the compartments.

2. The container of claim 1, wherein the compartments (22) are sufficiently deformable to assist removal of frozen milk from individual compartments.

3. The container of claim 1 or 2, further comprising a removable cover (32) for covering the base part.

4. The container of claim 3, wherein the base part (21) comprises an array of individual compartments (22) arranged in a plane, wherein the plane is parallel to a direction of attachment of the head to the breast pump.

5. The container of claim 3 or 4, wherein the removable cover (32) and the base part (21) each comprise compartment portions and channel portions which together define the channel arrangement and the plurality of individual compartments.

6. The container of any one of claims 1 to 5, wherein the head (26) and the base part (21) comprise a unitary piece.

7. The container of claim 6, wherein the unitary piece is silicone.

8. The container of any one of claims 1 to 5, wherein the head (26) is detachable from the base part (21).

9. The container of claim 8, wherein the base part (21) is silicone.

10. The container of any one of claims 1 to 3, wherein the base part (21) comprises a cup (60), and the container further comprises a divider (62) for dividing the cup into the individual compartments (22).

11. The container of claim 10, wherein the divider (62) is silicone.

12. The container of claim 10 or 11, wherein the compartments (22) comprise segments of a cylinder.

13. The container of claim 10, 11 or 12, wherein the head (26) forms a removable cover to the cup (60).

14. The container of claim 13, wherein the container further comprises a cap (64) for closing the opening (50) in the head.

15. The container of any one of claims 10 to 14, wherein the base part (21) is vertically stackable over an identical base part, such that a bottom of one base part functions as a closure lid for a base part beneath.
